# EUROPEAN PATENT APPLICATION

(11) **EP 4 706 666 A1**
(43) Date of publication of application: **11.03.2026**
(21) Application number: 24802691.6
(22) Date of filing: 12.04.2024
(51) Int. Cl.: A61K 33/08, A61P 19/02, A61P 29/00

(54) **USE OF MAGNESIUM HYDROXIDE IN PREPARATION OF DRUG FOR TREATING ARTHRALGIA**

(30) Priority: 05.05.2023 CN 202310496323; 05.05.2023 CN 202310497579
(71) Applicant: Xiangya Hospital, Central South University, Changsha, Hunan 410008 (CN)
(72) Inventor: LEI, Guanghua, Changsha, Hunan 410008 (CN); DENG, Caifeng, Changsha, Hunan 410008 (CN); ZENG, Chao, Changsha, Hunan 410008 (CN)
(74) Representative: Vesterinen, Jussi Tapio
(86) International application number: PCT/CN2024/087380
(87) International publication number: WO 2024/230405

(57) **Abstract**

Provided is application of magnesium hydroxide in preparation of a medicine for treating joint pain, relating to the field of pharmaceuticals. A rat experiment verifies that nano or micron-scale magnesium hydroxide treatment can effectively and permanently relieve the joint pain of OA rat, that is, the magnesium hydroxide can be used for preparing a medicine for treating joint pain. The medicine can greatly reduce a dosing frequency and further improve the compliance of a patient.

## Description

### CROSS-REFERENCE TO RELATED APPLICATION

This patent application claims the priority of Chinese Patent Application No. 202310496323.3 filed with the China National Intellectual Property Administration on May, 05, 2023 and entitled "Magnesium hydroxide nanoparticles for treating joint pain, preparation method therefor and application thereof", the disclosure of which is incorporated herein by reference in its entirety.

This patent application claims the priority of Chinese Patent Application No. 202310497579.6 filed with the China National Intellectual Property Administration on May, 05, 2023 entitled "Application of magnesium hydroxide in preparation of medicine for treating joint pain", the disclosure of which is incorporated herein by reference in its entirety.

### TECHNICAL FIELD

The present disclosure relates to the field of Provided is application of magnesium hydroxide in preparing a medicine for treating joint pain, relating to the field of medicines.

### BACKGROUND

Osteoarthritis (OA) is a degenerative disease caused by multiple factors, such as fibrosis, cracking, ulceration, and loss of articular cartilage, with joint pain as the main symptom. OA often affects sites such as knee joints, hip joints, spine, and hands, with pathological features mainly including degeneration and destruction of articular cartilage, subchondral bone sclerosis or cyst formation, osteophyte formation at joint margins, and synovitis. OA seriously affects joint function and quality of life of the patient, and it is found that OA, particularly symptomatic knee OA, is significantly associated with an increased risk of all-cause mortality. Unfortunately, there is no safe and effective treatment method in China and at abroad to delay the progress of OA.

Joint pain is the most common clinical manifestation of OA, so pain relief is the primary reason for the patient with OA to seek medical care. With the progress of the disease, the patient with OA often experiences persistent pain. Therefore, achieving long-acting and safe analgesia is of great significance for the patient with OA. At present, treatment methods for the patient with OA are primarily focused on joint pain relief. Although there is a wide variety of treatment methods, there are still numerous issues. For example, acetaminophen used to be the first-line drug to treat OA pain, but in recent years, high-quality research evidence shows that the acetaminophen is not only ineffective in relieving OA pain but also significantly increases the risk of gastrointestinal side effects compared with placebo. Oral non-steroidal anti-inflammatory drugs have a clear effect on relieving OA pain, but the cardiovascular and gastrointestinal side effects associated with chronic administration have raised safety concerns. Oral tramadol is recommended as a "first-line drug" by 2013 American Academy of Orthopaedic Surgeons and 2012 American College of Rheumatology Clinical Practice Guidelines for OA. However, in recent years, it has been found that oral tramadol can significantly increase all-cause mortality and the incidence of myocardial infarction and hip fracture compared with non-steroidal anti-inflammatory drugs (it has been written into 2019 American College of Rheumatology Clinical Practice Guidelines for OA and tramadol is no longer recommended as a "first-line drug"). Intra-articular injection of hormones is widely used and has obvious short-term pain relief effect, but there are some important problems to be solved urgently in the intra-articular injection of hormones, such as short duration of pain relief effect, insufficient effect and potential safety hazards. Therefore, there is an urgent need to explore a therapeutic drug for OA that offers significant efficacy, high safety profile, and provide long-acting analgesic effects.

The primary medical application of magnesium hydroxide is as an antacid. Clinically, the magnesium hydroxide is mainly used as a laxative for bowel cleansing prior to colonoscopy or intestinal surgery, effectively promoting the evacuation of fecal matter from the intestines. However, there have been no reports so far on the application of magnesium hydroxide as an active pharmaceutical ingredient for long-acting analgesia in osteoarthritis OA.

Based on this, the technical solution of the present disclosure is proposed.

### SUMMARY

To solve the problem in the prior art, the present disclosure provides application of magnesium hydroxide in preparation of a medicine for treating joint pain. During the research on pharmacological effects of magnesium hydroxide, the inventors find that intra-articular injection of magnesium hydroxide exhibits a good and long-lasting analgesic effect on OA.

The solution of the present disclosure is to provide application of magnesium hydroxide in preparation of a medicine for treating joint pain.

The application of magnesium hydroxide in preparation of a medicine for treating joint pain is provided.

Preferably, the magnesium hydroxide is applied in preparation of a medicine with long-acting joint analgesic effect.

Preferably, the joint pain is joint pain of osteoarthritis.

Preferably, cartilage injury is cartilage injury caused by joint degenerative change, inflammatory change, various types of traumata, or chronic wear.

Preferably, the magnesium hydroxide is nano-scale magnesium hydroxide particles.

Preferably, the magnesium hydroxide is micron-scale magnesium hydroxide particles.

Preferably, the magnesium hydroxide has an average particle size of 50 nm to 20 µm.

Preferably, the medicine is an injection preparation.

Preferably, the medicine is administrated through intra-articular injection.

Preferably, the medicine is a preparation prepared by taking magnesium hydroxide as a main medicine and adding medically acceptable carriers or additives.

Preferably, the carrier or additive is one or a combination of multiple selected from solvents, osmotic pressure regulators, pH regulators, stabilizers, suspending agents, surfactants, or preservatives.

The present disclosure has beneficial effects as follows.

A rat experiment verifies that nano or micron-scale magnesium hydroxide treatment can effectively and permanently relieve the joint pain of OA rat, that is, the magnesium hydroxide can be used for preparing a medicine for treating joint pain. The medicine can greatly reduce a dosing frequency and further improve the compliance of a patient.

### BRIEF DESCRIPTION OF THE DRAWINGS

To describe the technical solutions in the embodiments of the present disclosure or in the prior art more clearly, the following briefly introduces the accompanying drawings required for describing the embodiments. Apparently, the accompanying drawings in the following description show merely some embodiments of the present disclosure, and a person of ordinary skill in the art may still derive other drawings from these accompanying drawings without creative efforts.
FIG. 1 is a data plot of mechanical paw withdrawal threshold results for each group of rats;
FIG. 2 is a data plot of bipedal weight bearing difference results for each group of rats;
FIG. 3 is an HE (Hematoxylin-Eosin) staining diagram of knee joints of each group of rats.

### DETAILED DESCRIPTION OF THE EMBODIMENTS

To make the objective, technical solutions and advantages of the present disclosure more clearly, the technical solutions of the present disclosure are described in detail below. Apparently, the described embodiments are merely a part rather than all of the embodiments of the present disclosure. All other implementations obtained by a person of ordinary skill in the art based on the embodiments of the present disclosure without creative efforts shall fall within the scope of protection of the present disclosure.

### Embodiment 1

### Pharmacodynamic trial

Sodium iodoacetate is injected into a knee joint cavity of an SD rat to construct an OA rat model. When the knee joint of the rat swells and a pain threshold decreases significantly, the OA rat model is obtained. On the 7^{-th} day after modeling, OA model rats are treated with an equal volume (100 µL) of normal saline, a magnesium sulfate solution with a concentration of 0.35 mmol/mL (calculated as magnesium), a magnesium hydroxide nano-solution and a magnesium hydroxide suspension by intra-articular injection, which are designated as a normal saline group, a magnesium sulfate solution group, a nano-magnesium hydroxide group and a micron-magnesium hydroxide group, respectively.

A preparation method for the magnesium sulfate solution includes dissolving magnesium sulfate heptahydrate in the normal saline.

A preparation method for the magnesium hydroxide nano solution includes suspending nano-scale magnesium hydroxide (Aladdin, 99.8%) in the normal saline (an average particle size of the nano-scale magnesium hydroxide is 55 nm).

A preparation method for the magnesium hydroxide suspension includes treating a magnesium hydroxide bulk drug (Puli Pharmaceutical) by a jet mill, and then suspending the treated magnesium hydroxide in the normal saline (the average particle size of micron-scale magnesium hydroxide is 19.2 µm).

In addition, an equal volume of normal saline is injected into a knee joint cavity of the normal rat as control (designated as a normal group).

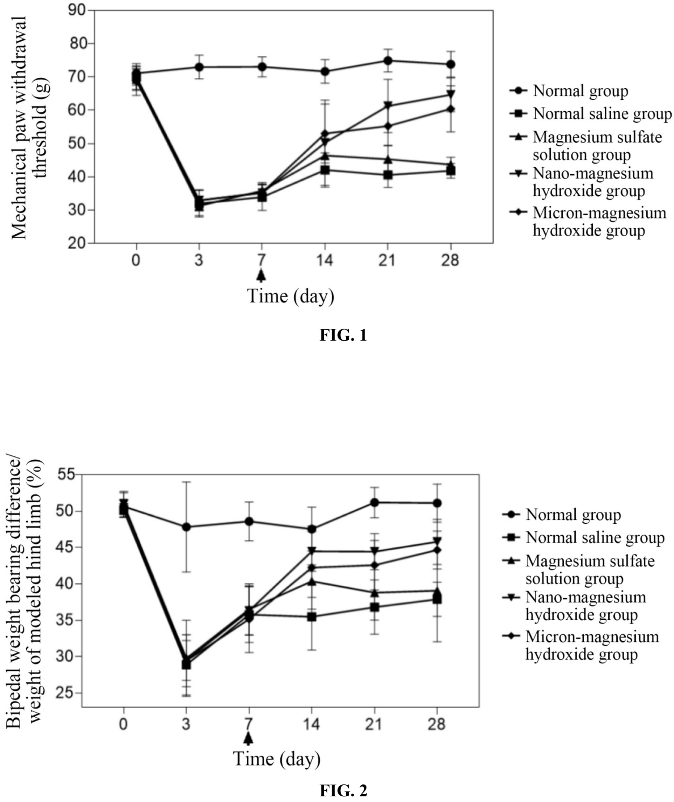

The medicine is administered once in total. From the first day of administration, mechanical paw withdrawal thresholds and bipedal weight bearing differences of each group of rats are measured every week. The results are shown in FIG. 1 and FIG. 2.

As can be learned from FIG. 1 and FIG. 2, the magnesium sulfate can relieve the joint pain of the OA rat to some extent, but the duration is short. The nano-or micron-scale magnesium hydroxide treatment can effectively and permanently relieve joint pain of the OA rat. These results show that the magnesium hydroxide provided by the present disclosure can be used for preparing a medicine for treating joint pain.

### Embodiment 2

### Pharmacodynamic trial

Sodium iodoacetate is injected into a knee joint cavity of an SD rat to construct an OA rat model. When the knee joint of the rat swells and a pain threshold decreases significantly, the OA rat model is obtained. On the 7^{-th} day after modeling, OA model rats are treated with an equal volume (100 µL) of normal saline, a magnesium sulfate solution with a concentration of 0.35 mmol/mL (calculated as magnesium), a magnesium hydroxide nano-solution and a magnesium hydroxide suspension by intra-articular injection, which are designated as a normal saline group, a magnesium sulfate solution group, a nano-magnesium hydroxide group and a micron-magnesium hydroxide group, respectively.

A preparation method for the magnesium sulfate solution includes dissolving magnesium sulfate heptahydrate in the normal saline.

A preparation method for the magnesium hydroxide nano solution includes suspending nano-scale magnesium hydroxide (Aladdin, 99.8%) in the normal saline.

A preparation method for the magnesium hydroxide suspension includes treating a magnesium hydroxide bulk drug (Puli Pharmaceutical) by a jet mill, and then suspending the treated magnesium hydroxide in the normal saline.

In addition, an equal volume of normal saline is injected into a knee joint cavity of the normal rat as control (designated as a normal group).

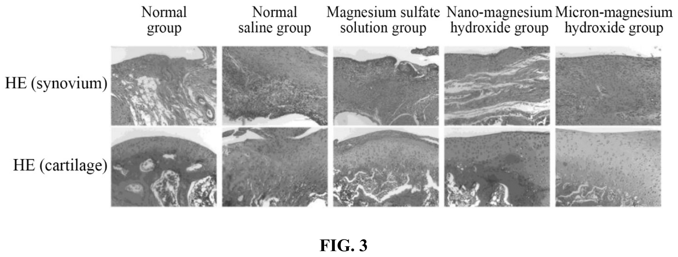

The medicine is administered once in total. After 4 weeks of administration, the treated knee joint is taken for paraffin section and stained with hematoxylin-eosin (HE staining). The results are shown in FIG. 3. The results show that the OA rat in the normal saline group has obvious synovitis, and the cartilage is seriously injured. After treatment with the magnesium sulfate solution, the synovitis and cartilage injury in the OA rat are relieved to some extent. Moreover, the synovitis of the OA rat treated with the nano-scale magnesium hydroxide and micron-scale magnesium hydroxide has been greatly improved, and the cartilage injury has also been effectively inhibited, and the state of the OA rat is close to that of the normal rat. These results show that the magnesium hydroxide can effectively inhibit synovitis and cartilage injury.

The foregoing is only the specific implementation of the present disclosure, but the scope of protection of the present disclosure is not limited thereto. Any variation or replacement that can be easily conceived by a person skilled in the art within the technical scope disclosed in the present disclosure shall fall within the scope of protection of the present disclosure. Therefore, the scope of protection of the present disclosure shall be subject to the appended claims.

## Claims

1. Application of magnesium hydroxide in preparation of a medicine for treating joint pain.

2. Application of magnesium hydroxide in preparation of a medicine for treating synovitis and cartilage injury.

3. The application according to claim 1, wherein application of the magnesium hydroxide in preparation of a medicine with long-acting joint analgesic effect is provided.

4. The application according to claim 1 or 3, wherein the joint pain is joint pain of osteoarthritis.

5. The application according to claim 2, wherein the cartilage injury is cartilage injury caused by joint degenerative change, inflammatory change, various types of traumata, or chronic wear.

6. The application according to claim 1 or 2, wherein the magnesium hydroxide is nano-scale magnesium hydroxide particles.

7. The application according to claim 1 or 2, wherein the magnesium hydroxide is micron-scale magnesium hydroxide particles.

8. The application according to claim 6 or 7, wherein the magnesium hydroxide has an average particle size of 50 nm to 20 µm.

9. The application according to claim 1 or 2, wherein the medicine is an injection preparation.

10. The application according to claim 9, wherein the medicine is administrated through intra-articular injection.

11. The application according to claim 10, wherein the medicine is a preparation prepared by taking magnesium hydroxide as a main medicine and adding medically acceptable carriers or additives.

12. The application according to claim 11, wherein the carrier or additive is one or a combination of multiple selected from solvents, osmotic pressure regulators, pH regulators, stabilizers, suspending agents, surfactants, or preservatives.
